# EUROPEAN PATENT SPECIFICATION

(11) **EP 2 608 088 B1**
(45) Date of publication and mention of the grant of the patent: **12.12.2018**
(21) Application number: 11194628.1
(22) Date of filing: 20.12.2011
(51) Int. Cl.: G06F 19/10, G01N 35/00, C12Q 1/6806, G16H 10/40

(54) **Improved method for nucleic acid analysis**
Verbessertes Verfahren zur Analyse von Nukleinsäure
Procédé amélioré pour analyse d'acide nucléique

(43) Date of publication of application: 26.06.2013
(73) Proprietor: F. Hoffmann-La Roche AG, 4070 Basel (CH); Roche Diagnostics GmbH, 68305 Mannheim (DE)
(72) Inventor: Frank, Paul, 6373 Ennetbuergen (CH); Roehrig, Sascha, 6006 Luzern (CH); Moertlseder, Alfred, 6331 Huenenberg (CH); Gisler, Andreas, 8800 Thalwil (CH)
(74) Representative: Herren, Barbara

(56) References cited:
- EP-A2- 0 693 560
- US-A1- 2005 009 070
- US-A1- 2009 143 995
- US-A1- 2010 173 394

## Description

### Background

The present application relates to an improved method for the analysis of nucleic acids in an automated system and to an analytical system for conducting such improved analysis.

Systems used in the field of nucleic acid analysis require processing of samples containing nucleic acids. Such processing involves the sorting of samples, the transfer of vessels, or of liquid samples and reagents from one vessel to another and analysis. For higher throughput, simultaneous processing is often performed with multiple consumables, such as pipette tips and single vessels or multi-well-plates. There is also an interest in analyzers which are integrated and which automatically carry out all or at least most steps necessary from sample preparation to the obtaining of the results of the analytical method.

### Summary of the invention

The invention is defined in independent method claim 1 and in independent system claim 5. Additional embodiments are defined in the dependent claims.

### General Description

Nucleic acid analysis has an ever increasing importance e.g. for detecting diseases, determining genetic conditions (genetic disorders and pre-dispositions) and for forensic purposes. When talking about nucleic acid analysis it is typically meant to detect the presence and maybe also the concentration of certain nucleic acids in a sample. For doing so, the sample material typically is treated to release nucleic acid material contained in the sample and often the nucleic acids to be detected are also purified. In view of the low amount of nucleic acids present in a sample and the often very limited amount of sample available, such detection is, however, not easy. Instead of increasing sensitivity of assays more and more, it has proven more useful to multiply the nucleic acid material contained in the original samples to make a proper detection of the presence or quantity of a nucleic acid feasible. A standard procedure for achieving such a multiplication - often called "amplification" - is the well-known polymerase chain reaction (PCR). For this amplification procedure the sample material (typically after some pre-treatment for releasing the nucleic acid and purifying it) is mixed with suitable reagents and then subjected to temperature cycles. Each temperature cycle - under optimal conditions - duplicates the amount of nucleic acid (NA) present in the sample. Detection of the presence and/or concentration of nucleic acid can be done at the end of amplification or during the amplification process as will be described below.
With the known instruments for amplification only samples which are tested for the same nucleic acid are subjected to the temperature cycles together. For amplification the samples are mixed with amplification reagents (to obtain amplification mixtures) and often disposed in the wells of a micro-well-plate (MWP).
The whole MWP is then subjected to the temperature cycles. This means the amplification mixtures are processed batch-wise. The number of wells in the MWP, determines the number of reaction mixtures which can be cycled together in one batch. In often used formats for MWPs are 48, 69, 356 and 1536 wells per plate.
If not enough samples are available which have to be tested for the same NA, then today two options exist. Either it is waited until further samples come in to fill the batch (i.e to complete open positions in the used MWP) or the batch is run with fewer samples as positions available in the plate (i.e. the MWP is cycled with empty wells). Option one increases the time until the result is available while option two wastes consumables. Both options therefore reduce testing efficiency.

It was found that the mentioned disadvantages (waiting for further samples or running incomplete batches) can be mitigated by a method and system which allows to run the analysis for different nucleic acids (i.e. different assays) within the same batch.
Such an improved method for nucleic acid analysis involves the steps of receiving sample tubes each containing a sample and receiving a test request for each sample specifying one or more assays to be conducted for said sample.

Obtaining one or more sample aliquots of each sample depending on if one or more assays are to be conducted with said sample.

For each of the sample aliquots it is then determined to which one or more test classes it belongs. This determination is made according to the test request which specifies the one or more assays to be conducted for a specific sample aliquot.

Sample aliquots for which assays are to be conducted which belong to the same test class are combined in a batch, said batch comprising at least one sample aliquot for which a first assay and a second sample aliquot for which a second assay different from the first assay is to be conducted.

The sample aliquots in that batch are then subjected to the same temperature profile for amplification of nucleic acids contained in the sample aliquots.

Detection of nucleic acids according to the first and second assays is then conducted what can take place during the amplification process or after completion of the amplification process.

The term "sample" as used herein is not only used for the original sample but also relates to a sample which has already been processed (pipetted, diluted, mixed with reagents, having been purified, having been amplified etc).

Sample aliquots are portions of a sample which are employed for testing. Sample aliquots are typically generated by pipetting a portion of a sample into a well where then further treatment is conducted. When two or more aliquots of a sample are needed it is for example possible to aspirate a volume of that sample and to discharge portions of that volume into two or more wells. The term sample aliquot is intended to cover also sample aliquots mixed with reagents and other fluids as it is typically necessary for assaying the sample aliquots.

Nucleic acid analysis nowadays is a powerful tool to determine infections (HIV, HCV etc), genetic diseases and predispositions as well as family relationships and the identification of persons for forensic purposes. Due to the minute amounts of nucleic acids in typical samples it is normally necessary to amplify the nucleic acids contained in a sample. Several methods are known for such amplification as the polymerase chain reaction (PCR), ligase chain reaction (LCR), transcription mediated analysis (TMA) and the like. These methods are well known in the art and are therefore not described in detail herein. A common requirement of such methods is, however, that the nucleic acids in the patient samples are amplified prior to detection. For such amplification a certain thermal treatment of the samples together with reagents is necessary (i.e. sample mixtures are generated). In automated systems where multiple samples are analyzed in parallel the thermal treatment of such amplification mixtures is done together (batch-wise).

The thermal treatment for amplification involves a certain temperature versus time profile which achieves an optimized and repeatable amplification of the nucleic acids. In short this is called the thermal profile. In case of PCR the thermal profile comprises multiple heating and cooling steps in a cyclic manner. An individual cycle comprises heating a reaction mixture from a baseline temperature (e.g. 40°C) to an upper level temperature (e.g. 70 °C) in a certain time, then keeping the upper level temperature, cooling to the baseline temperature and then keeping the baseline temperature for a predefined time. Assay development in in this field i.a. means to find a suitable thermal profile which achieves a sufficient and reproducible amplification of the nucleic acids which are to be detected. LCR and TMA require different but also well specified thermal profiles to obtain a reproducible amplification which in turn allows a standardized detection process.

A batch as used herein is a multitude of sample aliquots which are simultaneously subjected to the same thermal profile for nucleic acid amplification. This means the sample aliquots are grouped together in a way that the individual sample aliquots are subjected to the same thermal profile during amplification. In practice this is often done by pipetting the sample aliquots belonging to the same batch into wells of a microwell plate which is then treated with a thermal profile by a thermal unit. This means that each sample aliquot has its own well and the sample aliquots (i.e. fluids) are not mixed.

In addition to the same thermal treatment for NA amplification the sample aliquots belonging to one batch may be pipetted in parallel and may be processed simultaneously in other ways (as described herein below). This means sample aliquots may be batched together already prior to the nucleic acid amplification for conducting those steps. Batching together, however, does not mean that sample aliquots are mixed but the aliquots of a batch are each located in a separate well and being processed simultaneously. Such simultaneous processing at least comprises that the sample aliquots of a batch are subjected to the same thermal profile for nucleic acid amplification.

For conducting the mentioned assays the system comprises one or more analytical units or analyzers. It has to be understood that the analysis function for determining the presence and/or concentration of an analyte can be performed by a unit of an instrument which also performs other functions as e.g. pre-analytical steps or analysis can be performed by an instrument with more or less the sole purpose of analysis. In the context of the present application the term "analyzer" shell encompass all such embodiments.

An analyzer has a sensor which detects a signal identifying the presence and/or concentration of an analyte to be determined. In the field of nucleic acid analysis, the sensor is typically a fluorescence sensor that is able to detect fluorescence emitted by fluorescence labels which are bound to the analytes. In other embodiments the sensor can e.g. be a photomultiplier which detects radiation transmitted through a sample. For assaying multiple sample aliquots in parallel often imaging sensors are employed, e.g. CCD arrays. Such sensors are typically employed in combination with optical filters. An analyzer setup for detecting fluorescence from labeled nucleic acids during amplification is e.g. described in EP-B-1681555.

An analyte is a molecule for the detection of which analysis is conducted. Often the presence or absence of an analyte allows a medical doctor to render a diagnostic decision, i.e. to determine if the patient has a certain disease. In many cases such a diagnostic decision, however, cannot be drawn based on one analyte alone but two or more analytes have to be seen together or the result for an analyte has to be seen in the context the clinical situation of a patient.

Analytes according to the present application are nucleic acids and derivatives thereof. Derivatives of nucleic acids are inter alia nucleic acids to which a label is bound which allows detection of the nucleic acid. Such labels are e.g. fluorescence dyes.

A specific test for determining the presence and/or concentration of an analyte is called assay type. For the same analyte more than one type of assay may be available, as e.g. the case for detection of the analyte in different sample fluids (e.g. in blood and in urine). When such an assay type is applied for a particular sample it is said that the assay is conducted for that sample.

The specific measures which are taken to perform the detection of an analyte is named assay protocol. An assay protocol comprises the type and amount of reagents used, the processing steps, the consumables used and how the detection is performed.

As mentioned, prior to nucleic acid analysis often a sample treatment is necessary. Typical sample treatment steps are lysis of cells, purification and/or separation of nucleic acids to be detected, nucleic acid amplification and mixing with reagents for detection. As will be described herein later the nature of such sample treatment steps is pivotal for determining if the samples can be assigned to the same test class.

A system according to the present description has an input section into which sample tubes with sample therein are input. Often the sample tubes are located in racks each rack holding e.g. 5 sample tubes. The input into the input section in some embodiments is done manually but also automated input e.g. via a robotic arm or a conveyor is encompassed.

The sample tubes are containers (typically vials) for holding a sample. Such sample tubes have a closure for preventing loss of sample and preventing the contamination of sample during transport. The closure might have already been removed when a sample tube enters the input section or the system might be provided with a cap opener for opening or removing closures. In another embodiment the closures remain on the sample tubes and sample is withdrawn through the closure (e.g. by penetration with a steel needle). Sample tubes further have a tube identification affixed thereto. Such tube identification can be a barcode, an alphanumeric code, an electronic memory chip (e.g. an RFID tag), a magnetic code or the like. Reader for such codes are well known in the art (e.g. barcode reader and RFID reader).

The system may further comprise a control unit (CU) for controlling the analyzer. Such a control unit may be a separate unit or may be an integral part of an analytical instrument. The control unit is configured to control the analyzer in a way that the necessary steps for the assay protocols are conducted by the analyzer. That means the control unit is configured to e.g. instruct the analyzer to conduct certain pipetting steps to mix the sample with reagents or the control unit is configured to control the analyzer to incubate the sample mixtures for a certain time and so on. The control unit is configured to receive information from the data manager which test has to be done with a certain sample and based thereon to determine the steps the analyzer (and maybe also a sample preparation unit) has to perform. In certain embodiments the control unit might be integral with the data management unit or may be embodied by a common hardware.

The system further comprises a data management unit with a test request database. Such database allows to relate a sample tube identification with the assays to be conducted with the sample contained in the sample tube. The one or more analytical tests to be conducted with a particular sample are called a test request. The data management unit in many embodiments is connected to a LIS (laboratory information system) and/or a HIS (hospital information system). The data management unit (DMU) can be a unit within or co-located with an analyzer, e.g. it can be part of the control unit. Alternatively the DMU can be a unit remotely located from the analyzer, e.g. can be embodied in a computer connected via a network to the analyzer.

In a typical workflow a medical doctor decides on the analytical tests (assays) to be conducted for a patient and fills in a form on paper or on a computer. This test request is then transmitted to a medical technical assistant (MTA) for further processing. The term MTA as used herein shell not be limited to persons having undergone a formal education as MTA but is intended to also cover other persons as e.g. nurses and informally trained assistants performing the duties as herein described below.

The MTA obtains a sample (blood, urine, spinal fluid and the like) from the patient into one or more sample tubes according to the test request. The sample tube might be pre-labelled or might be labeled by the MTA in a way that a patient identifier is linked to the sample tube. For confidentiality and privacy this is typically done by a unique identifier affixed to the tube (the so called sample tube identification). The unique identifier is stored in a database (e.g. a database belonging to the above mentioned LIS) which links it to the name of the patient. A unique identifier is typically a barcode, a number or an alphanumeric code. The unique identifier does normally not show the patient name in plain language for confidentiality reasons.

If the test request has not already been input into the database by the medical doctor, the MTA will input it based on a form received. Accordingly the database has stored therein the relation of the unique identifier of the sample tube and the test request, i.e. the one or more analytical test (assays) to be conducted with the particular sample.

The database mentioned in this section might be the data management unit of the system of the present application or might be a LIS, a HIS or another database. In the latter cases the relation of the unique sample identification and the one or more tests to be conducted will be transmitted to the database of the system.

The test request for a sample determines the one or more analytical test (assays) to be conducted with the sample. Each of the assays is assigned to at least one test class. This assignment can be done by the data management unit, the control unit or another unit having computing capability. The assignment to at least one test class is done based on assignment rules which will be explained lateron.

The data management unit is configured to determine how many aliquots of a sample are needed (at least the number of different assays to be conducted for that sample) based on the test request and further configured to assign such samples to at least one test class. Normally the aliquots are not yet physically available at this time but the data management unit is planning for such aliquots and will instruct the control unit to produce the aliquots.

An assay requires a sequence of steps to be conducted which are defined in the assay protocol for that assay. An assay typically contains the following steps:
If lysis of sample is needed for the particular test: The lysis reagent(s) to be added to the sample for lysis, the volume of the one or more lysis reagents and the incubation period of sample with the one or more lysis reagents.

If isolation of nucleic acids is needed for the particular test: The type and volume of reagent(s) for isolating the nucleic acids to be detected, incubation periods, mixing or shaking actions, steps of separating the desired nucleic acids from other fluid/sample components and steps to set the nucleic acids to be detected free for further processing.

Amplification of nucleic acids to be detected: type and volume of amplification reagents, temperature profiles for amplifying the nucleic acids. In the case of PCR the reagents added for amplification are nucleotides and a temperature resistant polymerase. PCR further requires a temperature profile with alternating heating and cooling of the reaction mixtures. Such a temperature profile can be defined via the high and low temperatures, the times for which such high and low temperatures are kept as well as the ramping profiles, which is the heating or cooling rate typically given as Kelvin per second.

For detection of an analyte suitable reagents are added which in most cases provide a fluorescence signal upon illumination. Such detection reagents are e.g. labeled probes which bind to nucleic acids to be detected. Further reagents are intercalating dyes as SybGreen which intercalate into nucleic acid double strands. Various techniques are known in the art to render the analyte detectable, be it by labeling the analyte or by binding detectable entities.

In an assay protocol further the consumables to be used are specified. Such consumables are e.g. disposable pipette tips and containers (e.g. a microwell plate or individual vessels).

A detection unit is employed for conducting the detection. Such a detection unit comprises a light source to illuminate sample aliquots located in the wells of a batch and a detector to obtain signals from the sample aliquots which allow to determine if analytes are present in the sample aliquots. The detector is typically an optical detector which detects fluorescent light emitted by the sample aliquots upon illumination. Often detectors are employed which have a multitude of sensitive areas such that a two dimensional image of the batch can be recorded. The detection unit may further comprise various optical elements as lenses, filters, dichroitic mirrors and the like.

An evaluation unit is configured to evaluate signals obtained from the detector to determine if analytes are present in the sample aliquots. The evaluation unit typically is a computing unit which is configured to compare signals from the detector with stored reference values or thresholds. Prior to this the evaluation unit may be configured to correct received signals for offsets and artifacts and may even analyze image information to extract signals of certain areas which represent signals from the analytes. The evaluation unit may be further configured to record signals from the detector over time. Such intensity over time information may be analyzed by fitting to curves or other mathematical algorithms to determine the presence and / or concentration of analytes in the sample aliquots.

A test class according to the present description indicates which sample aliquots can be processed together in a batch. The test class assigned to a particular sample aliquot is chosen according to at least the thermal profile to be employed for the nucleic acid amplification (incubation temperature, time profile, number of amplification steps). This means sample aliquots are assigned to the same test class if their thermal profile for amplification is the same, even though different assays will be conducted with sample aliquots of the same batch. It has to be understood that in addition to the thermal treatment for amplification also other steps of the assay may be conducted together with sample aliquots of the same batch.

The same test class is assigned to two sample aliquots if the thermal profile to be employed for the nucleic acid amplification is the same for both sample aliquots.

As a further requirement for assigning the same test class to two sample aliquots it can be requested that
the sample processing steps are the same for the two sample aliquots and/or that both sample aliquots require the same consumables for processing.

It is, however, advantageous if the same test class is only assigned to two sample aliquots if all three criteria (thermal profile for amplification, sample processing steps, employed consumables) are the same.

The database mentioned above, however, does not need to check these criteria at each time a test request is entered into the system. As mentioned above an assay protocol already contains the details of the thermal profile for NA amplification and hence it can be determined on the level of an assay to which test class it belongs to. Or in other words the types of assays available for a system can be checked for the thermal profile they require for NA amplification and hence assigned to a test class. That means assays requiring the same thermal profile for NA amplification are assigned to the same test class while assays having different thermal profiles are assigned to different test classes. This means the number of test classes equals the number of assay types or is lower. Advantages of the process described herein, however, can only be achieved if the number of test classes is lower than the number of assay types. Only in this case sample aliquots for which different assays are to be conducted can be batched together for amplification.

It is convenient to assign each assay type to a respective test class and to store the correlation of assay types with test classes in a lookup table. A sample aliquot then can be assigned to a test class by checking the test order which type of assay is to be conducted for that sample aliquot and looking up the respective test class in the lookup table for that type of assay. Such a lookup table may have only the first two columns of the below table while the other columns are shown for explanation only:

| Assay type | Test Class | bottom /high level temp | Reagent |
|---|---|---|---|
| A | I | 40 / 70 | M |
| B | II | 45 / 80 | N |
| C | I | 40 / 70 | O |
| D | II | 45 / 80 | P |

It can be seen that sample aliquots which are tested with assays A and C belong to the same test class and accordingly can be batched together for amplification. Similarly samples with assays B and D can be combined together in a batch for amplification while assay A samples cannot be combined with assay B or assay D samples in the same batch. It further can be seen that in this case two test classes have been defined according to the two different thermal profiles needed to conduct the assays.

The term batching as used herein does not means that sample aliquots are mixed together to obtain a fluid mixture. It rather means that the sample aliquots are combined in a way that they can be processed simultaneously for at least the amplification process of the assays. This can e.g. be achieved by putting sample aliquots into separate wells of the same microwell plate which is then treated with a thermal profile such that all sample aliquots are subjected to the same thermal profile.

Sample aliquots belonging to the same test class can be sorted into the same batch even if the assays to be conducted with the sample aliquots are different. This has a number of advantages compared to methods or systems where only samples requiring the same tests are grouped together for processing and analysis. When sample aliquots with different test requests are grouped together (batched together) the batches are filled faster and hence the waiting time for a sample aliquot to be analyzed can be reduced. Further the efficiency of the analytical system can be enhanced when mixed batches (batches containing samples aliquots for which different assays are to be conducted) are allowed as processing of incomplete batches or less filled batches can be avoided. It has, however, to be understood that the method does not at all require that a batch is completely filled (i.e. all available positions in that batch are filled) or that only mixed batches are allowed. The advantages are already available if the system has the freedom to fill the batches with sample aliquots having different tests requests.

### Short description of the figures:

Figure 1 shows a system with hardware units and logical units for generating batches of samples based on test classes.
Figure 2 shows the optical setup of a system for real-time detection.
Figure 3 shows the elements of a system for generating batches of sample aliquots
Figure 4 shows a segmented thermal unit with a microwell plate containing sample aliquots.

### Detailed description of the figures:

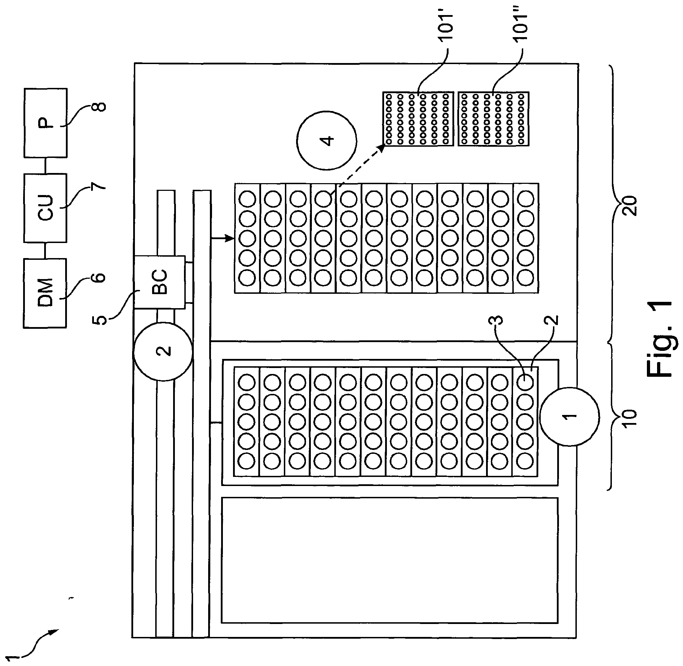

Figure 1 shows a system for generating batches of samples. Racks (2) with sample tubes (3) are input into an input section (10) of the system (1) . This can be done manually or automatically by e.g. a robotic rack handler. The racks are conveyed from the input section into a sample pipetting section (20). During this movement the barcodes on the sample tubes are read by a barcode reader (5). The barcode reader sends the read data directly or via other units to a data management unit (6). The data management unit may be integrated into the system or may be a unit co-located with it. Alternatively the data management unit may be remote from the system, e.g. a computerized unit connected with the instrument via a network. The data management unit (6) determines a test class for each sample or sample aliquot based on the test request. This is done by checking the test request which assays have to be conducted and then checking in a lookup table to which test class the assays belong.

The control unit (7) receives data from the data management unit which assay or assays have to be conducted with a certain sample. The control unit then determines which steps have to be performed by the analyzer to conduct such assays. The control unit in particular controls a pipettor unit (8) such that sample aliquots belonging to the same test class are pipetted from the sample tubes into wells of the same microwell plate. Figure 1 shows two microwell plates. Sample aliquots belonging to test class I are pipetted into the wells of microwell plate 101', while sample aliquots belonging to test class II are pipetted into microwell plate 101". Microwell plates 101' and 101" are thereafter processed differently. A number of steps concerning pipetting, employed reagents, consumables, incubation, separation of nucleic acids and the like may be the same for both assays or different. Important, however, is that sample aliquots belonging to the same test class are subjected to the same thermal profile for amplification of nucleic acids.

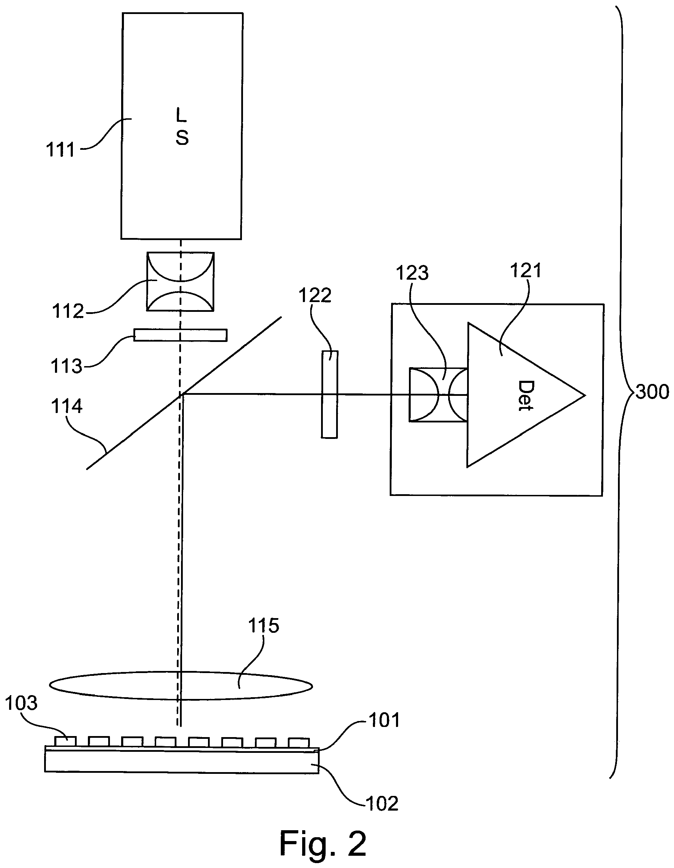

Figure 2 shows a system for so-called real-time detection. A microwell plate (101) with multiple microwells (103) containing sample aliquots and reagents is located in a thermal unit (102) and is analyzed with a detection unit (300). The thermal unit can be realized by a mount made of metal (typically silver or copper) which is thermally connected to a Peltier element. The Peltier element is controlled by the control unit to heat and cool the mount - and therefore also the sample mixtures in the microwell plate - according to a pre-defined temperature profile. Such temperature profile comprises multiple cycles of heating the mount to a desired temperature (e.g. 90° Celsius) and to cool it to a specified temperature (e.g. 40 ° Celsius) in order to perform nucleic acid amplification. While the sample mixtures are subject to the temperature profile, a fluorescence detection unit measures the fluorescence emitted from the wells of the microwell plate. The fluorescence detection unit as shown comprises a light source (e.g. a LED lamp or a Xenon lamp) (111) for illuminating the sample mixtures and a detector (121) which detects fluorescence emitted from the wells. The radiation of the light source is guided through optical elements (112) for focusing the light and a filter (113) which transmits a wavelength band of light suitable to trigger fluorescence emission of fluorescence labels contained in the wells. The light then transmits a dichroitic mirror (114) and is distributed by a lens or lens system (115) into the wells. Fluorescence radiation emitted from the wells is collected by the lens system (115) and reflected by the dichroitic mirror (114). This means the dichroitic mirror is chosen to transmit the excitation wavelength and to reflect the emitted light. The emitted radiation is filtered by a second filter (122) and guided with optical elements (123) onto the detector (121). The detector is typically a CCD (charge coupled device) detector which generates an image of the emission intensities. This image is then evaluated to determine if the respective analytes are contained within wells of the microwell plate. More details about the setup and function of such a real-time detection system can be e.g. found in EP1681555.

The microwell plates 101' and 101 " according to figure 1 can be analyzed with dedicated analyzers according to figure 2 or the microwell plates 101' and 101" can be analyzed with the same analyzer subsequently.

According to the concept described herein all sample aliquots belonging to the same test class are, however subjected to the same temperature profile to perform nucleic acid amplification. In principle two embodiments are possible. A uniform thermal unit heats all the wells of a microwell plate to the same temperature at a time and therefore subjects all the wells to the same temperature profile. This means that in the same microwell plate only assays are conducted which belong to the same test class. Alternatively, however, a segmented thermal unit can be employed which has segments (maybe two or four segments) which can run different thermal profiles. This means while one segment e.g. cycles from 40 to 70 °C, another segment of the thermal unit cycles from 45 to 80 °C. This means some microwells of the same microwell plate are subjected to a first thermal profile while others are subjected to a second thermal profile. In case of a segmented thermal unit all sample aliquots located in wells which are heated by a particular segment belong to the same test class while overall the microwell plate may contain sample aliquots belonging to different test classes.

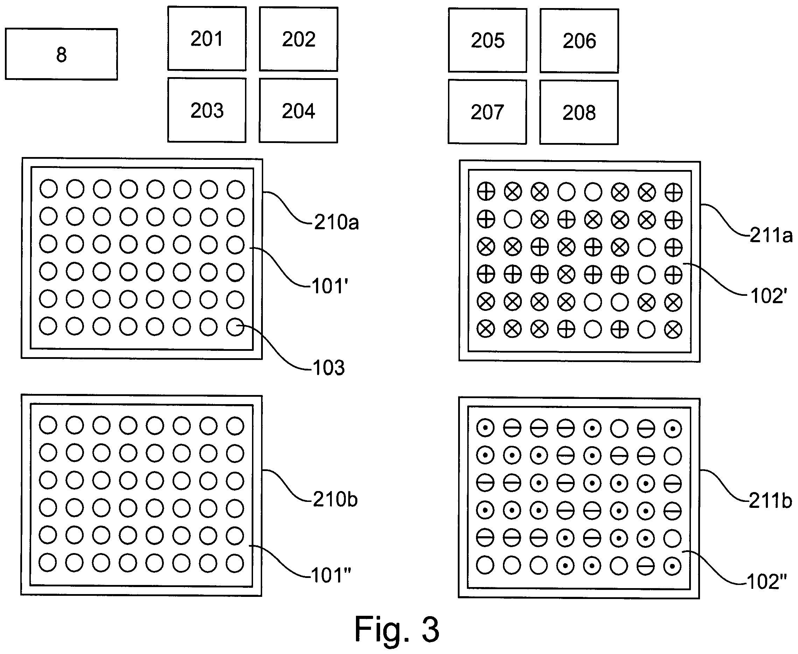

Figure 3 shows a system for analyzing samples for the presence and/or concentration of nucleic acids. The depicted microwell plates 101' and 101" have been filled with sample fluid according to figure 1. The two microwell plates 101', 101" each contain sample aliquots for which at least two different assays have to be conducted, that is some of the sample aliquots in microwell plate 101' wells will be tested for the presence of analyte A, while others are tested for the presence of analyte B.

Pipettor 8 pipettes lysis reagent from container 201 into the wells of the plates 101' and 101". These plates are located in the thermal units 210a and 210b where they are incubated to release nucleic acids from the sample fluid in the presence of lysis reagent. Units 201a and 201b further have the function to purify released nucleic acids. For this a suspension of magnetically attractable microparticles is pipetted from container 202 into the wells of the plates. The released nucleic acids bind to the microparticles which are then separated on the inner walls of the wells by magnetic forces and the fluid is removed from the wells with the pipettor. The microparticles further can be suspended in washing fluid from container 203, again be separated on the inner walls of the wells and fluid removed from the wells. Finally the microparticles are suspended in elution fluid from container 204 to release nucleic acids bound to the microparticles. The particles are then separated on the inner well walls and fluid containing the purified nucleic acids is withdrawn with the pipettor from the wells and deposed in the wells of microwell plates 102' and 102". Reagents 205, 206, 207, 208 are then added to the purified nucleic acid containing wells according to the assay protocol. Sample aliquots to be tested for analyte A are mixed with reagent 205, those to be tested for B are mixed with reagent 206 and further reagent 207 for C and reagent 208 for analyte D. Thermal cycling is then conducted with the thermal units 211a and 211b. These units further have detection units as shown in figure 2 to monitor the amplification of nucleic acids during the thermal cycles and to evaluate the presence and/or concentration of nucleic acids in the wells.

In the depicted embodiment sample aliquots for which assays A and B have to be conducted are pipetted into plate 201' while sample aliquots which are tested for C and D are present in plate 202'. Each of the thermal units 201a and 201b can run a different temperature profile while all wells within the same unit are subjected to the same temperature profile (i.e. the thermal units in figure 3 are uniform). Accordingly assays A and B have to have the same temperature profile as both tests are conducted in the same unit 210a. This means, assays A and B are only assigned to the same test class if they are compatible with the same incubation temperature profile.

Assays A, B, C and D are belonging to two different test classes according to the following table:

| Assay | Test Class | bottom /high level temp | reagent |
|---|---|---|---|
| A | I | 40 / 70 | M |
| B | II | 45 / 80 | N |
| C | I | 40 / 70 | O |
| D | II | 45 / 80 | P |

Sample aliquots which are tested according to assay A (denoted by +) and those which are tested according to assay C (denoted by X) are batched together in microwell plate 102'. Microwell plate 102' is treated in thermal unit 211a with thermocycling between 40 and 70 °C.

Sample aliquots which are tested according to assay B (denoted by •) and those which are tested according to assay D (denoted by -) are batched together in microwell plate 102". Microwell plate 102" is treated in thermal unit 211b with thermocycling between 45 and 80 °C.

As can be seen, better usage can be made of the microwell plates when sample aliquots for different assays are batched together compared to automated systems where only sample aliquots which are tested according to a single assay are loaded into the same microwell plate. It can also be seen that some wells of the microwell plate can be left empty (no denotion) if not enough sample aliquots are present to fill the microwell plate.

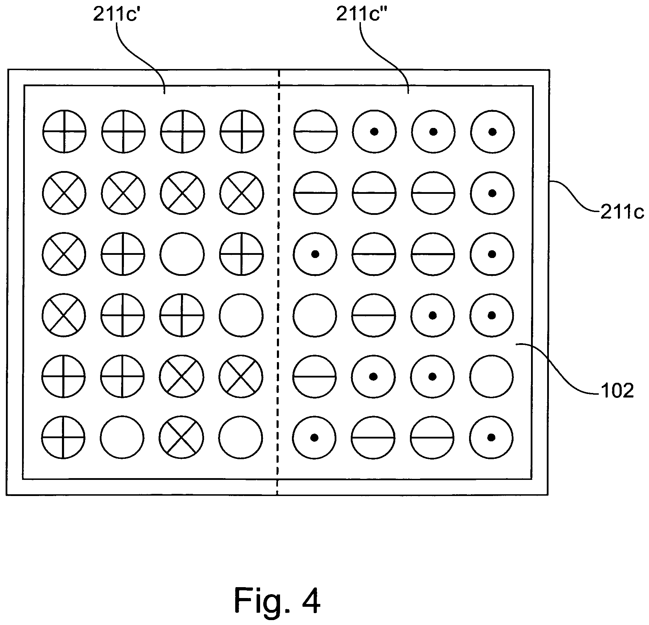

Figure 4 shows a segmented thermal unit 211c which has two areas (211c', 211c") which can be temperature regulated independently from one another. Segmented thermal units are e.g. described in US2005/0009070 and WO2008/030914. With such thermal units it is possible to perform different thermal profiles with sample mixtures located in wells thermally coupled to the two segments. As can be seen thermal treatment according to assays A and C is conducted in segment 211c' while thermal treatment according to assays B and D is conducted in segment 211c". With such a segmented thermal unit it is hence possible to make good usage of microwell plates and achieve a high throughput if only relatively few assays of each type are to be conducted. For an artisan it is well conceivable to design and employ thermal units with more than two segments (e.g. three or four).

## Claims

1. Method for nucleic acid analysis involving the steps of
- receiving sample tubes each containing a sample,
- receiving a test request for each sample, the test request specifying one or more assays to be conducted for the sample,
- obtaining one or more sample aliquots of each sample, each aliquot corresponding to an assay to be conducted according to the test request ,
- assigning each of the one or more sample aliquots of each sample to a test class according to the assay which is to be conducted for that sample aliquot, wherein the test class to which a particular sample aliquot is assigned is chosen at least according to a thermal profile to be employed for the assay to be conducted for that particular sample aliquot,
- combining sample aliquots belonging to the same test class into a batch in a multiwell plate, the batch comprising a sample aliquot for which a first assay is to be conducted and a sample aliquot for which a second, different assay is to be conducted, the first assay and the second, different assay employing at least a same thermal profile,
- adding reagents to the sample aliquots in the batch,
- simultaneously amplifying nucleic acids contained in the sample aliquots in the batch using the same thermal profile,
- detecting signals from the sample aliquots in the batch to determine the presence and/or concentration of nucleic acids in the sample aliquots.

2. Method according to claim 1, wherein
assigning involves a check of a lookup table which shows a corresponding test class for an assay to be conducted.

3. Method according to claim 1, further comprising assigning aliquots to the same test class if
a.) the sample processing steps of the first assay and the second, different assay are identical or
b.) the same consumables are employed in both the first assay and the second, different assay.

4. Method according to claim 1, wherein conducting an analysis involves detection of fluorescence light from the sample aliquots.

5. System (1) for analyzing nucleic acid containing samples, comprising
- a sample reception unit (10) configured to receive sample tubes (3) each containing a sample,
- a reader (5) configured to read identifications on the sample tubes,
- a data management unit (6) configured to receive test requests for samples, the test requests specifying assays to be conducted on the samples, the data management unit being further configured to receive sample tube identifications from the reader, wherein the data management unit is configured to determine how many aliquots of a sample are necessary for each of the samples according to their corresponding test requests, further configured to assign the sample aliquots to test classes, wherein the test class assignment is at least based on a thermal profile used by an assay to which the sample aliquot will be subjected, and further configured to provide such assignment to a control unit (7), wherein the control unit is configured to control a pipettor (8) to pipette sample aliquots having the same test class assigned thereto into a batch, the batch comprising wells in a multiwell plate (101', 101"), and wherein each sample aliquot having the same test class assigned thereto is pipetted into a separate well of the multiwell plate (101', 101"),
- the same or a further pipettor for pipetting reagents into the wells of the multiwell plate (101', 101"),
- a thermal unit (102, 210a, 210b) for subjecting the multiwell plate (101', 101") to the thermal profile for amplifying nucleic acids,
- a detection unit (300) configured to detect signals from the wells of the multiwell plate (101', 101"),
- an evaluation unit configured to determine the presence and / or quantity of nucleic acids in the wells of the multiwell plate (101', 101").

6. System according to claim 5 further comprising a purification unit configured to purify nucleic acids contained in the wells of the multiwell plate (101', 101").

7. System according to claim 5, wherein the data management unit has stored therein a lookup table, the data management unit being configured to use said lookup table to assign test classes to the sample aliquots according to the assay to which the sample aliquot is to be subjected.

8. System according to claim 5, wherein the thermal unit has two or more segments which are configured to perform different thermal profiles.

9. System according to claim 5, wherein the detection system is a fluorescence detection system comprising an illumination unit for illuminating sample aliquots located in the sample wells and further comprising a detector for detecting fluorescent light emitted from the sample wells.

10. System according to claim 5, wherein the data management unit is configured to receive the sample orders from a laboratory information system (LIS).

11. System according to claim 5, wherein the data management unit is further configured to assign sample aliquots used for a first assay and a second, different assay to the same test class if in addition to the first and second, different assay using the same thermal profile;
a.) the sample processing steps of the first assay and the second, different assay are identical or
b.) the same consumables are employed in both the first assay and the second, different assay.

## Patentansprüche

1. Verfahren zur Analyse von Nukleinsäure, umfassend die folgenden Schritte
- Erhalten von Probenröhrchen, die jeweils eine Probe enthalten,
- Erhalten eines Testauftrags für jede Probe, wobei der Testauftrag einen oder mehrere Assays spezifiziert, die für die Probe durchgeführt werden sollen,
- Gewinnen eines oder mehrerer Probenaliquote jeder Probe, wobei jedes Aliquot einem Assay entspricht, der gemäß dem Testauftrag durchgeführt werden soll,
- Zuordnen jedes der ein oder mehreren Probenaliquote jeder Probe zu einer Testklasse gemäß dem Assay, der für dieses Probenaliquot durchgeführt werden soll, wobei die Testklasse, der ein bestimmtes Probenaliquot zugeordnet wird, mindestens gemäß einem Wärmeprofil ausgewählt wird, das für den für dieses bestimmte Probenaliquot durchzuführenden Assay eingesetzt werden soll,
- Vereinigen der Probenaliquote, die zur selben Testklasse gehören, zu einem Satz in einer Multiwell-Platte, wobei der Satz ein Probenaliquot, für das ein erster Assay durchgeführt werden soll, und ein Probenaliquot, für das ein zweiter, unterschiedlicher Assay durchgeführt werden soll, umfasst, wobei der erste Assay und der zweite, unterschiedliche Assay mindestens ein gleiches Wärmeprofil einsetzen,
- Zugeben von Reagenzien zu den Probenaliquoten im Satz,
- gleichzeitiges Amplifizieren von Nukleinsäuren, die in den Probenaliquoten im Satz enthalten sind, unter Verwendung des gleichen Wärmeprofils,
- Nachweisen von Signalen von den Probenaliquoten im Satz, um die Gegenwart und/oder Konzentration von Nukleinsäuren in den Probenaliquoten zu bestimmen.

2. Verfahren nach Anspruch 1, wobei
das Zuordnen eine Überprüfung einer Nachschlagetabelle umfasst, die eine entsprechende Testklasse für einen durchzuführenden Assay zeigt.

3. Verfahren nach Anspruch 1, ferner umfassend das Zuordnen von Aliquoten zur gleichen Testklasse, wenn
a.) die Probenverarbeitungsschritte des ersten Assays und des zweiten, unterschiedlichen Assays identisch sind oder
b.) die gleichen Verbrauchsmaterialien sowohl im ersten Assay als auch im zweiten, unterschiedlichen Assay eingesetzt werden.

4. Verfahren nach Anspruch 1, wobei das Durchführen einer Analyse den Nachweis von fluoreszierendem Licht von den Probenaliquoten umfasst.

5. System (1) zur Analyse von Nukleinsäure enthaltenden Proben, umfassend
- eine Probenaufnahmeeinheit (10), die konfiguriert ist, um Probenröhrchen (3) aufzunehmen, die jeweils eine Probe enthalten,
- eine Lesevorrichtung (5), die konfiguriert ist, um Kennzeichnungen auf den Probenröhrchen abzulesen,
- eine Datenverwaltungseinheit (6), die konfiguriert ist, um Testaufträge für Proben zu empfangen, wobei die Testaufträge Assays spezifizieren, die an den Proben durchgeführt werden sollen, wobei die Datenverwaltungseinheit ferner konfiguriert ist, um Probenröhrchenkennzeichnungen von der Lesevorrichtung zu empfangen, wobei die Datenverwaltungseinheit konfiguriert ist, um für jede der Proben zu bestimmen, wie viele Aliquote einer Probe gemäß dem entsprechenden Testauftrag notwendig sind, und ferner konfiguriert ist, um die Probenaliquote Testklassen zuzuordnen, wobei die Testklassenzuordnung mindestens auf einem Wärmeprofil basiert, das von einem Assay, dem das Probenaliquot ausgesetzt wird, verwendet wird, und ferner konfiguriert ist, um eine derartige Zuordnung einer Steuereinheit (7) bereitzustellen, wobei die Steuereinheit konfiguriert ist, um eine Pipettierhilfe (8) zu steuern, um Probenaliquote mit der gleichen zugeordneten Testklasse in einen Satz zu pipettieren, wobei der Satz Vertiefungen in einer Multiwell-Platte (101', 101") umfasst, und wobei jedes Probenaliquot mit der gleichen zugeordneten Testklasse in eine getrennte Vertiefung der Multiwell-Platte (101', 101") pipettiert wird,
- die gleiche oder eine weitere Pipettierhilfe zum Pipettieren von Reagenzien in die Vertiefungen der Multiwell-Platte (101', 101"),
- eine Wärmeeinheit (102, 210a, 210b) zum Aussetzen der Multiwell-Platte (101', 101") einem Wärmeprofil, um Nukleinsäuren zu amplifizieren,
- eine Nachweiseinheit (300), die konfiguriert ist, um Signale von den Vertiefungen der Multiwell-Platte (101', 101") nachzuweisen,
- eine Bewertungseinheit, die konfiguriert ist, um die Gegenwart und/oder Menge von Nukleinsäuren in den Vertiefungen der Multiwell-Platte (101', 101") zu bestimmen.

6. System nach Anspruch 5, ferner umfassend eine Reinigungseinheit, die konfiguriert ist, um Nukleinsäuren zu reinigen, die in den Vertiefungen der Multiwell-Platte (101', 101") enthalten sind.

7. System nach Anspruch 5, wobei in der Datenverwaltungseinheit eine Nachschlagetabelle gespeichert ist, wobei die Datenverwaltungseinheit konfiguriert ist, um die Nachschlagetabelle zu verwenden, um den Probenaliquoten Testklassen gemäß dem Assay, dem das Probenaliquot ausgesetzt werden soll, zuzuordnen.

8. System nach Anspruch 5, wobei die Wärmeeinheit zwei oder mehr Segmente aufweist, die konfiguriert sind, um verschiedene Wärmeprofile auszuführen.

9. System nach Anspruch 5, wobei das Nachweissystem ein Fluoreszenznachweissystem ist, das eine Beleuchtungseinheit zum Beleuchten von Probenaliquoten, die sich in den Probenvertiefungen befinden, umfasst und ferner einen Detektor zum Nachweisen von fluoreszierendem Licht umfasst, das von den Probenvertiefungen emittiert wird.

10. System nach Anspruch 5, wobei die Datenverwaltungseinheit konfiguriert ist, um die Probenbestellungen von einem Laborinformationssystem (LIS) zu empfangen.

11. System nach Anspruch 5, wobei die Datenverwaltungseinheit ferner konfiguriert ist, um Probenaliquote, die für einen ersten Assay und für einen zweiten, unterschiedlichen Assay verwendet werden, der gleichen Testklasse zuzuordnen, wenn zusätzlich dazu, dass im ersten und zweiten, unterschiedlichen Assay das gleiche Wärmeprofil verwendet wird;
a.) die Probenverarbeitungsschritte des ersten Assays und des zweiten, unterschiedlichen Assays identisch sind oder
b.) die gleichen Verbrauchsmaterialien sowohl im ersten Assay als auch im zweiten, unterschiedlichen Assay eingesetzt werden.

## Revendications

1. Procédé pour l'analyse d'acides nucléiques impliquant les étapes de
- réception de tubes d'échantillon contenant chacun un échantillon,
- réception d'une demande de test pour chaque échantillon, la demande de test spécifiant un ou plusieurs dosages à réaliser pour l'échantillon,
- obtention d'une ou plusieurs aliquotes d'échantillon de chaque échantillon, chaque aliquote correspondant à un dosage à réaliser selon la demande de test,
- attribution de chacune de la ou des aliquotes d'échantillon de chaque échantillon à une classe de test selon le dosage qui doit être réalisé pour cette aliquote d'échantillon, dans lequel la classe de test à laquelle est attribuée une aliquote d'échantillon particulière est choisie au moins selon un profil thermique à utiliser pour le dosage à réaliser pour cette aliquote d'échantillon particulière,
- combinaison des aliquotes d'échantillon appartenant à la même classe de test en un lot dans une plaque multi-puits, le lot comprenant une aliquote d'échantillon pour laquelle un premier dosage doit être réalisé et une aliquote d'échantillon pour laquelle un second dosage différent doit être réalisé, le premier dosage et le second dosage différent utilisant au moins un même profil thermique,
- ajout de réactifs aux aliquotes d'échantillon dans le lot,
- amplification simultanée des acides nucléiques contenus dans les aliquotes d'échantillon dans le lot en utilisant le même profil thermique,
- détection de signaux en provenance des aliquotes d'échantillon dans le lot pour déterminer la présence et/ou la concentration des acides nucléiques dans les aliquotes d'échantillon.

2. Procédé selon la revendication 1, dans lequel
l'attribution implique une vérification d'une table de conversion qui montre une classe de test correspondante pour un dosage à réaliser.

3. Procédé selon la revendication 1, comprenant en outre l'attribution d'aliquotes à la même classe de tests si
a.) les étapes de traitement d'échantillon du premier dosage et du second dosage différent sont identiques ou
b.) les mêmes consommables sont utilisés à la fois dans le premier dosage et le second dosage différent.

4. Procédé selon la revendication 1, dans lequel la mise en oeuvre d'une analyse implique la détection d'une lumière de fluorescence en provenance des aliquotes d'échantillon.

5. Système (1) permettant d'analyser des échantillons contenant des acides nucléiques, comprenant
- une unité de réception d'échantillons (10) configurée pour recevoir des tubes d'échantillon (3) contenant chacun un échantillon,
- un lecteur (5) configuré pour lire les identifications sur les tubes d'échantillon,
- une unité de gestion de données (6) configurée pour recevoir des demandes de test pour les échantillons, les demandes de test spécifiant les dosages à réaliser sur les échantillons, l'unité de gestion de données étant en outre configurée pour recevoir les identifications des tubes d'échantillon en provenance du lecteur, dans lequel l'unité de gestion de données est configurée pour déterminer combien d'aliquotes d'un échantillon sont nécessaires pour chacun des échantillons selon leurs demandes de test correspondantes, en outre configurée pour attribuer les aliquotes d'échantillon aux classes de test, dans lequel l'attribution de classes de tests est au moins basée sur un profil thermique utilisé par un dosage auquel sera soumise l'aliquote d'échantillon, et en outre configurée pour fournir une telle attribution à une unité de commande (7), dans lequel l'unité de commande est configurée pour commander à une pipette (8) de pipetter les aliquotes d'échantillon auxquelles a été attribuée la même classe de test dans un lot, le lot comprenant des puits dans une plaque multi-puits (101', 101"), et dans lequel chaque aliquote d'échantillon à laquelle est attribuée la même classe de tests est pipettée dans un puits séparé de la plaque multi-puits (101', 101"),
- la même pipette ou une autre pipette pour pipetter des réactifs dans les puits de la plaque multi-puits (101', 101"),
- une unité thermique (102, 210a, 210b) pour soumettre la plaque multi-puits (101', 101") au profil thermique pour amplifier les acides nucléiques,
- une unité de détection (300) configurée pour détecter les signaux en provenance des puits de la plaque multi-puits (101', 101"),
- une unité d'évaluation configurée pour déterminer la présence et/ou la quantité d'acides nucléiques dans les puits de la plaque multi-puits (101', 101").

6. Système selon la revendication 5, comprenant en outre une unité de purification configurée pour purifier les acides nucléiques contenus dans les puits de la plaque multi-puits (101', 101").

7. Système selon la revendication 5, dans lequel l'unité de gestion de données a stocké en son sein une table de conversion, l'unité de gestion de données étant configurée pour utiliser ladite table de conversion pour attribuer les classes de test aux aliquotes d'échantillon selon le dosage auquel doit être soumise l'aliquote d'échantillon.

8. Système selon la revendication 5, dans lequel l'unité thermique a deux segments ou plus qui sont configurés pour effectuer des profils thermiques différents.

9. Système selon la revendication 5, dans lequel le système de détection est un système de détection de fluorescence comprenant une unité d'éclairage permettant d'éclairer les aliquotes d'échantillon situées dans les puits d'échantillon et comprenant en outre un détecteur permettant de détecter la lumière fluorescente émise à partir des puits d'échantillon.

10. Système selon la revendication 5, dans lequel l'unité de gestion de données est configurée pour recevoir les commandes d'échantillons en provenance d'un système d'informations de laboratoire (SIL).

11. Système selon la revendication 5, dans lequel l'unité de gestion de données est en outre configurée pour attribuer les aliquotes d'échantillon utilisées pour un premier dosage et un second dosage différent à la même classe de test si, en plus du premier et du second dosage différent utilisant le même profil thermique ;
a.) les étapes de traitement d'échantillon du premier dosage et du second dosage différent sont identiques ou
b.) les mêmes consommables sont utilisés à la fois dans le premier dosage et le second dosage différent.
